# EUROPEAN PATENT APPLICATION

(11) **EP 3 492 583 A1**
(43) Date of publication of application: **05.06.2019**
(21) Application number: 17833979.2
(22) Date of filing: 05.07.2017
(51) Int. Cl.: C12N 5/071, C12Q 1/02, G01N 33/15, G01N 33/50, A61K 35/36, A61L 27/60, A61P 17/00

(54) **CELL LAMINATE, METHOD FOR PRODUCING CELL LAMINATE, METHOD FOR EVALUATING EPIDERMAL PROLIFERATION OR DIFFERENTIATION, METHOD FOR EVALUATING TEST SUBSTANCE, AND BIOLOGICAL IMPLANTATION MATERIAL**

(30) Priority: 29.07.2016 JP 2016150296
(71) Applicant: FUJIFILM Corporation, Tokyo 106-8620 (JP)
(72) Inventor: MURAGUCHI, Taichi, Ashigarakami-gun Kanagawa 258-8577 (JP); TASHIRO, Tomoko, Ashigarakami-gun Kanagawa 258-8577 (JP)
(74) Representative: Hoffmann Eitle
(86) International application number: PCT/JP2017/024647
(87) International publication number: WO 2018/020970

(57) **Abstract**

Disclosed herein are a cell laminate including an epidermal layer in which specific gene-deficient epidermal keratinocytes are laminated; a method for producing a cell laminate, including a step of culturing specific gene-deficient epidermal keratinocytes by air-liquid interface culture; and applications thereof

## Description

### BACKGROUND OF THE INVENTION

### 1. Field of the Invention

The present disclosure relates to a cell laminate, a method for producing a cell laminate, a method for evaluating epidermal proliferation or differentiation, a method for evaluating a test substance, and a biological implantation material.

### 2. Description of the Related Art

With the progress of cell culture technology, it has become possible to artificially reconstruct the skin of a living body to produce artificial skin having a structure and a function similar to those of the skin of a living body.

The reconstructed artificial skin is used as a biological implantation material for the treatment of, for example, burns or wounds. In addition, the reconstructed artificial skin is also used as an evaluation system for elucidating the structure or function of the skin, or an evaluation system for developing a pharmaceutical product, a cosmetic product, or the like, for which a variety of techniques have been proposed so far.

For example, JP5458259B discloses a cell laminate for use as a bioassay system, including a first cell layer, a layer formed of non-enzyme treated type IV collagen, and a second cell layer laminated in this order, in which the second cell layer is of a collagen gel containing fibroblasts and the second cell layer is of epidermal keratinocytes.

JP5228246B discloses a three-dimensional disease skin reconstruct obtained by three-dimensionally culturing a transgenic epidermal basal cell, into which a nucleic acid encoding epimorphin is introduced in an expressible manner, on a support.

### SUMMARY OF THE INVENTION

Various endogenous genes are involved in the structure and function of the skin. However, the cell laminate of JP5458259B did not pay attention to the endogenous gene of the skin. In addition, the three-dimensional disease skin reconstruct of JP5228246B is obtained by culturing a cell into which a foreign gene has been introduced, not focusing on endogenous genes of the skin.

An object according to an embodiment of the present invention is to provide a novel cell laminate obtained by culturing specific gene-deficient epidermal keratinocytes and a method for producing the same. Another object according to the embodiment of the present invention is to provide a method for evaluating epidermal proliferation or differentiation, a method for evaluating a test substance, and a biological implantation material, to each of which a cell laminate and a method for producing the same are applied.

Specific means for achieving the foregoing objects includes the following aspects.
<1> A cell laminate comprising an epidermal layer in which specific gene-deficient epidermal keratinocytes are laminated.
<2> The cell laminate according to <1>, in which the epidermal keratinocyte is a human epidermal keratinocyte.
<3> The cell laminate according to <1> or <2>, further comprising a support layer.
<4> The cell laminate according to <3>, in which the support layer is a layer including collagen and at least one of mesenchymal cells or mesenchymal stem cells.
<5> The cell laminate according to any one of <1> to <4>, in which the specific gene includes a cell membrane receptor gene.
<6> The cell laminate according to any one of <1> to <5>, in which the specific gene includes an insulin-like growth factor 1 receptor gene.
<7> A method for producing a cell laminate, comprising a step of culturing specific gene-deficient epidermal keratinocytes by air-liquid interface culture.
<8> The method for producing a cell laminate according to <7>, in which the epidermal keratinocyte is a human epidermal keratinocyte.
<9> The method for producing a cell laminate according to <7> or <8>, further comprising a step of deleting the specific gene of the epidermal keratinocyte using a site-specific nuclease.
<10> The method for producing a cell laminate according to any one of <7> to <9>, in which the step of culturing specific gene-deficient epidermal keratinocytes by air-liquid interface culture includes culturing the specific gene-deficient epidermal keratinocytes on a support layer by air-liquid interface culture.
<11> The method for producing a cell laminate according to <10>, in which the support layer is a layer including collagen and at least one of mesenchymal cells or mesenchymal stem cells.
<12> The method for producing a cell laminate according to any one of <7> to <11>, in which the specific gene includes a cell membrane receptor gene.
<13> The method for producing a cell laminate according to any one of <7> to <12>, in which the specific gene includes an insulin-like growth factor 1 receptor gene.
<14> A method for evaluating epidermal proliferation or differentiation, comprising a step of culturing specific gene-deficient epidermal keratinocytes by air-liquid interface culture.
<15> A method for evaluating a test substance, comprising a step of culturing specific gene-deficient epidermal keratinocytes in the presence of a test substance by air-liquid interface culture.
<16> A method for evaluating a test substance, comprising a step of bringing a test substance into contact with the cell laminate according to any one of <1> to <6>.
<17> A biological implantation material comprising the cell laminate according to any one of <1> to <6>.

According to the present disclosure, it is possible to provide a novel cell laminate obtained by culturing specific gene-deficient epidermal keratinocytes and a method for producing the same. Further, according to the present disclosure, it is possible to provide a method for evaluating epidermal proliferation or differentiation, a method for evaluating a test substance, and a biological implantation material, to each of which a cell laminate and a method for producing the same are applied.

### BRIEF DESCRIPTION OF THE DRAWINGS

Fig. 1 is a diagram schematically showing an example of a culturing step in a method for producing a cell laminate of the present disclosure.
Fig. 2 is a diagram schematically showing another example of the culturing step in the method for producing a cell laminate of the present disclosure.
Fig. 3 is a diagram showing the position of a target sequence on an insulin-like growth factor-1 receptor (IGF-1R) gene in the case where the IGF-1R gene of a HaCaT cell is deleted by a clustered regularly interspaced short palindromic repeats-CRISPR-associated proteins 9 (CRISPER-CAS9) method.
Fig. 4 is a diagram showing the results of Cell assay after introducing a plasmid vector for CRISPER-CAS9 into a HaCaT cell, followed by cloning.
Fig. 5 is a diagram showing the results of Western blotting using a Cell assay-positive strain after cloning.
Fig. 6 is a diagram showing the results of cell immunostaining using a Cell assay-positive strain after cloning.
Fig. 7 is a diagram showing the results of Western blotting after passage of an IGF-1R gene-deficient HaCaT cell.
Fig. 8 is a diagram showing the results of confirming a sequence of a region containing the target sequence of an IGF-1R gene for an IGF-1R gene-deficient HaCaT cell.
Fig. 9 is a diagram showing a hematoxylin/eosin (HE) stained image of a cell laminate produced by culturing an IGF-1R gene-deficient HaCaT cell on a support by air-liquid interface culture.
Fig. 10 is a diagram showing an average thickness of epidermal layers of cell laminates produced by culturing an IGF-1R gene-deficient HaCaT cell on a support by air-liquid interface culture.
Fig. 11 is a diagram showing a HE stained image of a cell laminate produced by culturing an IGF-1R gene-deficient HaCaT cell on a support layer (collagen gel in which fibroblasts are embedded) by air-liquid interface culture.

### DESCRIPTION OF THE PREFERRED EMBODIMENTS

Hereinafter, the cell laminate, the method for producing a cell laminate, the method for evaluating epidermal proliferation or differentiation, the method for evaluating a test substance, and the biological implantation material of the present disclosure will be described in detail. However, the present invention is not limited to the following embodiments at all and can be carried out with appropriate modification within the scope of the object of the present invention.

The numerical range indicated by using "to" in the present specification means a range including numerical values described before and after "to" as a minimum value and a maximum value, respectively.

In the numerical ranges described in a stepwise manner in the present specification, the upper limit value or the lower limit value described in a certain numerical range may be replaced with the upper limit value or the lower limit value of the numerical range in other stepwise description. In addition, in the numerical ranges described in the present specification, the upper limit value or the lower limit value described in a certain numerical range may be replaced with the values shown in the Examples.

Unless otherwise specified, the concentration of each component in the present specification means the total concentration of a plurality of substances in the case where there are a plurality of substances corresponding to each component.

The term "layer" in the present specification includes the case where a layer is formed only in a part of the region in addition to the case where a layer is formed in the whole region in the case of observing the region where the layer exists.

The term "step" in the present specification includes not only an independent step but also a step in which the intended purpose of this step can be achieved even in the case where the step cannot be clearly distinguished from other steps.

### <Cell laminate>

The cell laminate of the present disclosure includes an epidermal layer on which specific gene-deficient epidermal keratinocytes (hereinafter, also referred to as "gene-deficient epidermal keratinocytes") are laminated. The cell laminate of the present disclosure may have only an epidermal layer or may further include layers other than an epidermal layer such as a support layer described below.

### (Epidermal layer)

The epidermal layer is a layer in which at least gene-deficient epidermal keratinocytes are laminated. Here, the "lamination" may be such that gene-deficient epidermal keratinocytes are piled up, and it is not required that the division of the layers can be clearly confirmed.

Examples of epidermal keratinocytes include cells derived from mammals. Examples of mammals include primates such as humans, rhesus monkeys, marmosets, orangutans, and chimpanzees; rodents such as mice, rats, hamsters, and guinea pigs; and rabbits, pigs, cows, goats, horses, sheep, minks, dogs, and cats. Among them, primate epidermal keratinocytes are preferred, and human epidermal keratinocytes are more preferred.

The type of the human epidermal keratinocyte is not particularly limited, and examples thereof include HaCaT cells (J. Invest. Dermatol., 1999, 112 (3): 343-353), human dermal keratinocytes (HDKs) (Cancer Sci., 2007, 98: 147-154), and normal human epidermal keratinocytes (NHEKs). Among these, HaCaT cells are preferable from the viewpoint of relatively uniform cell properties irrespective of the number of passages, easy handling, and normal cell differentiation state.

The epidermal keratinocyte may be a non-immortalized cell or an immortalized cell. The non-immortalized cell refers to a cell whose proliferation stops due to a finite number of cell divisions. On the other hand, the immortalized cell refers to a cell that does not stop proliferation even in the case where cell division is repeated, that is, a cell that has acquired an infinite autonomous replication ability. Immortalization of a cell can be carried out by introducing an immortalizing gene such as a simian virus (SV) 40 T antigen gene, a telomerase reverse transcriptase (TERT) gene, or a human papilloma virus (HPV) E6-E7 gene into the cell. In addition, the immortalization of a cell may occur occasionally. For example, the above-mentioned HaCaT cell is an immortalized cell that has been naturally immortalized without introducing an immortalizing gene.

There is no particular limitation on the type of a specific gene lacking in epidermal keratinocytes. Examples of the specific gene include cell membrane receptor genes and skin disease causative genes. Examples of cell membrane receptors include a tyrosine kinase receptor such as insulin-like growth factor-1 receptor (IGF-1R) or epidermal growth factor receptor (EGF-R); a G protein-coupled receptor; a guanylate cyclase receptor; and an ion channel type receptor. The specific genes lacking in epidermal keratinocytes may be of one type or of two or more types.

In one aspect, the specific gene includes a cell membrane receptor gene (for example, an IGF-1R gene). Since it is thought that the cell membrane receptor gene plays an important role in the homeostasis of the epidermal layer of the living body, use of epidermal keratinocytes deficient in the cell membrane receptor gene makes it possible to obtain a novel cell laminate which is different from cell laminates in the related art. For example, as shown in the Examples which will be described later, a cell laminate in which the epidermal layer is thinned can be obtained by culturing IGF-1R gene-deficient epidermal keratinocytes by air-liquid interface culture.

In another aspect, the specific gene includes a skin disease causative gene. As will be described later, a cell laminate produced using epidermal keratinocytes deficient in a skin disease causative gene can be used as a biological implantation material for the treatment of skin diseases.

A gene-deficient epidermal keratinocyte can be obtained by deleting a specific gene of the epidermal keratinocyte. From the viewpoint of specifically deleting a specific gene, a method using a site-specific nuclease is preferable as a method of deleting a specific gene of the epidermal keratinocyte. Examples of the method using a site-specific nuclease include a CRISPR-Cas9 method, a transcription activator-like effector nucleases (TALENs: registered trademark) method, and a zinc finger nucleases (ZFNs) method. In one aspect, the gene-deficient epidermal keratinocyte is obtained by *in vitro* deletion of a specific gene of the epidermal keratinocyte.

Among them, the CRISPR-Cas9 method is preferable from the viewpoint of convenience and the ability to simultaneously edit a plurality of sites on genomic DNA. The principle of the CRISPR-Cas9 method is as follows. That is, in the CRISPR-Cas9 method, Cas9 nuclease and guide RNA (gRNA) are introduced into a cell. The gRNA includes CRISPR RNA (crRNA) having a base sequence complementary to a target sequence (for example, a sequence on a gene to be deleted) and trans-activating crRNA (tracrRNA), and binds to genomic DNA to thereby induce cleavage of the target sequence by the Cas9 nuclease. Mutation of the target sequence is caused during the repair process of the cleaved genomic DNA, and as a result, it becomes possible to lose the gene.

For example, the following methods (a) to (d) are known as the method for introducing Cas9 nuclease and gRNA into a cell. Among them, the method (a) is preferable from the viewpoint of convenience and introduction efficiency.
(a) a method in which DNA encoding Cas9 nuclease and DNA encoding gRNA are introduced into a cell through a plasmid vector.
(b) a method in which DNA encoding Cas9 nuclease is introduced into a cell through a viral vector and DNA encoding gRNA is directly introduced into the cell.
(c) a method in which RNA encoding Cas9 nuclease and gRNA are constructed by *in vitro* transcription and are directly introduced into a cell.
(d) a method in which a recombinant protein of Cas9 nuclease and gRNA constructed by *in vitro* transcription are directly introduced into a cell.

The epidermal layer may contain one type of gene-deficient epidermal keratinocyte or may contain two or more types of gene-deficient epidermal keratinocytes. As an aspect containing two or more types of gene-deficient epidermal keratinocytes, for example, an aspect containing two or more types of gene-deficient epidermal keratinocytes which have the same cell type of epidermal keratinocyte but have different types of specific genes to be deleted; an aspect containing two or more types of gene-deficient epidermal keratinocytes which have the same type of the specific gene to be deleted but have different cell types of the epidermal keratinocytes; and an aspect containing two or more types of gene-deficient epidermal keratinocytes which have different cell types of the epidermal keratinocytes and different types of the specific genes to be deleted.

The epidermal layer may contain cells other than the gene-deficient epidermal keratinocytes. Examples of other cells include melanocytes and cells of the immune system.

The number of cells other than the gene-deficient epidermal keratinocytes may be 20% or less, 15% or less, or 10% or less with respect to the total number of cells in the epidermal layer.

Further, the epidermal layer may be a layer in which a four-layer structure of a basal layer, a stratum spinosum, a granular layer, and a horny cell layer is confirmed, or may be a layer in which only a part of the four layers is confirmed. The method of confirming each of the above layers is not particularly limited, and the layers can be confirmed by a known method. In general, each layer can be confirmed by HE staining of the epidermal layer and confirmation of characteristic structure and position information under an optical microscope.

### (Support layer)

The cell laminate of the present disclosure may further include a support layer. The support layer is not particularly limited as long as it can support the epidermal layer and form a cell laminate.

The support layer may be a membrane filter from the viewpoint that preparation and handling at the time of culture of gene-deficient epidermal keratinocytes by air-liquid interface culture are facilitated. Examples of the material for the membrane filter include polyethylene terephthalate (PET), polycarbonate, and polytetrafluoroethylene (PTFE).

Further, from the viewpoint that the structure and function of the cell laminate can be brought closer to the skin of a living body, the support layer is preferably a layer containing collagen and at least one of mesenchymal cells or mesenchymal stem cells (hereinafter, also referred to as "collagen-containing layer"). The collagen-containing layer corresponds to the dermal layer of the skin. For the homeostasis of the epidermal layer of a living body, intercellular signaling plays an important role in addition to growth factors and nutrients supplied from the dermal layer. The cell laminate of the present disclosure further includes a collagen-containing layer, so that the structure and function of the cell laminate can be brought closer to the skin of a living body.

Examples of mesenchymal cells and mesenchymal stem cells contained in the collagen-containing layer include cells derived from mammals. Examples of mammals include primates such as humans, rhesus monkeys, marmosets, orangutans, and chimpanzees; rodents such as mice, rats, hamsters, and guinea pigs; and rabbits, pigs, cows, goats, horses, sheep, minks, dogs, and cats. Among them, primate cells are preferred and human cells are more preferred.

The type of mesenchymal cells is not particularly limited, and examples thereof include fibroblasts, adipocytes, cardiomyocytes, bone cells, chondrocytes, and tenocytes. Of these, fibroblasts are preferable from the viewpoint of bringing the cell laminate closer to the skin of a living body.

In addition, the type of mesenchymal stem cells is not particularly limited and examples thereof include mesenchymal stem cells derived from bone marrow, adipose tissue, placental tissue, umbilical cord tissue, dental pulp, or the like. Among them, bone marrow-derived mesenchymal stem cells are preferable from the viewpoint of maintenance of undifferentiation. The fact that the mesenchymal stem cell is derived from bone marrow can be determined by analyzing the cluster of differentiation (CD) antigen of the cell by, for example, cell immunostaining, flow cytometer, or Western blotting.

Mesenchymal cells and mesenchymal stem cells may be non-immortalized cells or immortalized cells. From the viewpoint of bringing the proliferation state and differentiation state of the epidermis closer to the epidermis of a living body, mesenchymal cells and mesenchymal stem cells are preferably non-immortalized cells.

The collagen-containing layer may contain one type of mesenchymal cells or may contain two or more types of mesenchymal cells. In addition, the collagen-containing layer may contain one type of mesenchymal stem cells or may contain two or more types of mesenchymal stem cells.

The collagen contained in the collagen-containing layer is not particularly limited, and for example, fibrillar collagen that gels can be mentioned. Examples of the fibrillar collagen include type I collagen, type II collagen, type III collagen, type V collagen, and type XI collagen, among which type I collagen or type III collagen is preferable, and type I collagen is more preferable. The collagen may be acid-solubilized collagen. Acid-solubilized type I collagen is more preferable as the collagen.

The origin of collagen is not particularly limited, and examples thereof include collagen derived from human, cow, horse, pig, mouse, rat, or the like.

The collagen-containing layer may contain one type of collagen or may contain two or more types of collagen.

The collagen-containing layer may be, for example, a collagen gel in which at least one of mesenchymal cells or mesenchymal stem cells is embedded. Such a collagen gel can be constructed, for example, by gelling a collagen solution in which collagen and at least one of mesenchymal cells or mesenchymal stem cells are added to a medium. The medium is not particularly limited and may be, for example, a medium for a cell laminate which is used in air-liquid interface culture that will be described later. The density of the collagen gel is preferably, for example, about 0.1 mg/mL to 100 mg/mL.

The collagen-containing layer may contain cells other than mesenchymal cells and mesenchymal stem cells. Examples of other cells include cells of the immune system.

The cell density per cm² of the bottom area of the collagen-containing layer is, for example, preferably 1×10⁵ cells/cm² or more, more preferably 2.5×10⁵ cells/cm² or more, still more preferably 5×10⁵ cells/cm² or more, and particularly preferably 1×10⁶ cells/cm² or more. The number of cells in the collagen-containing layer can be measured by a conventional method using a cell counter or a counting chamber. In addition, the number of cells can be estimated based on the calibration curve also by an MTT colorimetric assay. The MTT assay is a colorimetric determination method which measures the enzymatic activity of reducing 3-(4,5-dimethyl-thiazol-2-yl)-2,5-diphenyltetrazolium bromide (MTT) or a similar dye to a formazan dye (violet).

### (Other layers)

The cell laminate of the present disclosure may further include layers other than the epidermal layer and the support layer. For example, the cell laminate of the present disclosure may include a layer formed of non-enzyme treated type IV collagen (see JP5458259B) between the epidermal layer and the support layer.

### <Method for producing cell laminate>

The method for producing a cell laminate of the present disclosure includes a step of culturing specific gene-deficient epidermal keratinocytes (that is, the above-mentioned gene-deficient epidermal keratinocytes) by air-liquid interface culture (hereinafter, also referred to as "culturing step"). The epidermal keratinocytes are preferably human epidermal keratinocytes.

The method for producing a cell laminate of the present disclosure may further include a step of deleting a specific gene of an epidermal keratinocyte using a site-specific nuclease. As a method using a site-specific nuclease, for example, the above-mentioned CRISPR-Cas9 method can be mentioned. In a certain aspect, the specific gene includes a cell membrane receptor gene (for example, an IGF-1R gene). In one aspect, the step of deleting a specific gene of an epidermal keratinocyte using a site-specific nuclease is carried out *in vitro.* As shown in the Examples which will be described later, a cell laminate in which the epidermal layer is thinned can be obtained by culturing IGF-1R gene-deficient epidermal keratinocytes by air-liquid interface culture.

### (Medium)

The medium for use in the culturing step (hereinafter, also referred to as "medium for a cell laminate") may be, for example, a medium in which serum is added to a base medium. The medium for a cell laminate may further contain CaCl₂, an ascorbic acid derivative, an antibiotic, and/or a pH buffering agent.

Examples of the base medium include a Dulbecco's Modified Eagle Medium (DMEM), an Eagle's Minimum Essential Medium (EMEM), a Minimum Essential Medium Alpha modification (MEMα), a Roswell Park Memorial Institute 1640 (RPMI 1640) medium, and a Ham's F12 (HamF12) medium. The base media may be used alone or in combination of two or more thereof.

The serum may be, for example, fetal bovine serum (FBS). The concentration of serum in the medium for a cell laminate is preferably, for example, 5 v/v% to 20 v/v%.

The medium for a cell laminate preferably contains CaCl₂. By incorporation of CaCl₂ in the medium for a cell laminate, the differentiation of epidermal keratinocytes tends to be promoted. Further, calcium ions are also necessary ions for intercellular adhesion.

In the case where the medium for a cell laminate contains CaCl₂, the concentration of calcium in the medium for a cell laminate is preferably, for example, 0.5 mmol/L to 5 mmol/L and more preferably 1 mmol/L to 3 mmol/L.

The medium for a cell laminate preferably contains an ascorbic acid derivative. By incorporation of the ascorbic acid derivative in the medium for a cell laminate, epidermal keratinocytes are activated, resulting in a tendency to promote cell differentiation.

Examples of the ascorbic acid derivative include ascorbic acid, sodium ascorbate, potassium ascorbate, calcium ascorbate, L-ascorbic acid phosphate ester, magnesium ascorbyl phosphate, sodium ascorbyl phosphate, ascorbyl sulfate, ascorbyl sulfate disodium salt, and ascorbyl-2-glucoside.

In the case where the medium for a cell laminate contains an ascorbic acid derivative, the concentration of the ascorbic acid derivative in the medium for a cell laminate is preferably, for example, 0.01 w/w% to 1 w/w%.

The medium for a cell laminate may contain an antibiotic. Examples of the antibiotic include penicillin, streptomycin, neomycin, amphotericin B, kanamycin, and gentamicin.

The medium for a cell laminate may contain a pH buffering agent. Examples of the pH buffering agent include sodium hydrogen carbonate, calcium chloride, sodium dihydrogen phosphate, 2-[4-(2-hydroxyethyl)-1-piperazinyl]ethanesulfonic acid (HEPES), and 3-morpholinopropane-1-sulfonic acid (MOPS).

The pH of the medium for a cell laminate is preferably, for example, pH 6.8 to pH 7.6 and more preferably pH 7.0 to pH 7.4.

### (Culturing step)

There is no particular limitation on the method for culturing the gene-deficient epidermal keratinocytes by air-liquid interface culture to produce a cell laminate. For example, a known method for producing artificial skin using epidermal keratinocytes can be adopted. The air-liquid interface culture of gene-deficient epidermal keratinocytes may be carried out on a support such as a cell culture insert made of polycarbonate or may be carried out on the above-described support layer.

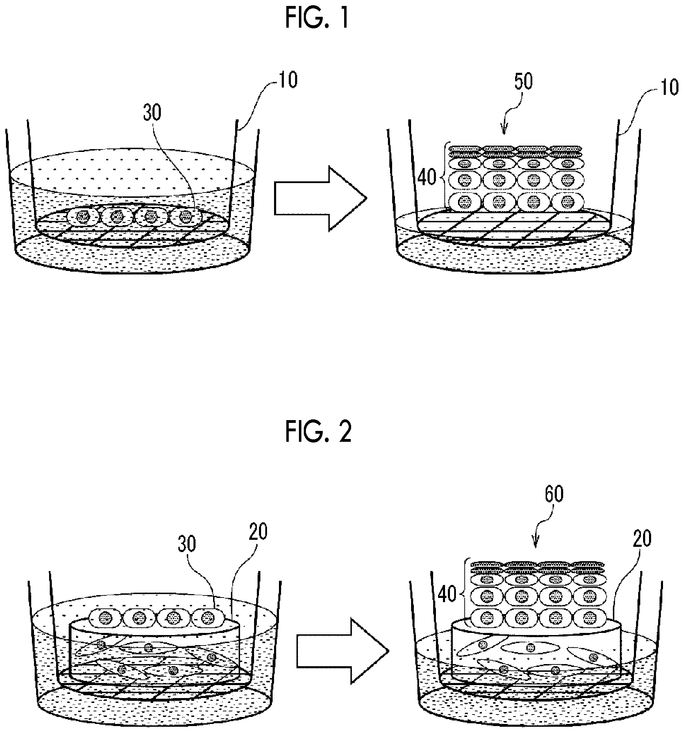

An example of the culturing step is shown in Fig. 1. First, gene-deficient epidermal keratinocytes 30 are seeded on a support 10 and cultured to form a layer of gene-deficient epidermal keratinocytes. Subsequently, the medium on the layer of the gene-deficient epidermal keratinocytes is removed to form an air-liquid interface and the culture is continued, whereby it is possible to obtain a cell laminate 50 including an epidermal layer 40 in which gene-deficient epidermal keratinocytes are laminated.

Another example of the culturing step is shown in Fig. 2. First, a collagen gel in which at least one of mesenchymal cells or mesenchymal stem cells is embedded is prepared as a support layer 20. Subsequently, gene-deficient epidermal keratinocytes 30 are seeded on the support layer 20 and cultured to form a layer of gene-deficient epidermal keratinocytes. Subsequently, the medium on the layer of the gene-deficient epidermal keratinocytes is removed to form an air-liquid interface, and the culture is continued, whereby it is possible to obtain a cell laminate 60 including the support layer 20 and the epidermal layer 40 in which gene-deficient epidermal keratinocytes are laminated.

There is no particular restriction on the number of passages of gene-deficient epidermal keratinocytes seeded on the support 10 or the support layer 20. In the case where the gene-deficient epidermal keratinocytes are non-immortalized cells, the number of passages of the gene-deficient epidermal keratinocytes is preferably, for example, 10 passages or less, more preferably 8 passages or less, and still more preferably 5 passages or less.

General conditions can be adopted as culture conditions for air-liquid interface culture. For example, conditions of 37°C and 5 v/v% CO₂ can be adopted.

The number of days of culture by air-liquid interface culture is preferably, for example, 10 days or more and more preferably 11 days or more. By setting the number of days of culture to 10 days or more, the state of differentiation of the epidermal layer tends to be sufficient. The upper limit of the number of days of culture is not particularly limited, and it is generally within 30 days and preferably within 25 days.

In the above description, only the gene-deficient epidermal keratinocytes are seeded, but other cells such as melanocytes may be seeded together with gene-deficient epidermal keratinocytes.

### <Method for evaluating epidermal proliferation or differentiation>

The method for evaluating epidermal proliferation or differentiation of the present disclosure includes a step of culturing specific gene-deficient epidermal keratinocytes (that is, the above-mentioned gene-deficient epidermal keratinocytes) by air-liquid interface culture. The epidermal proliferation or differentiation can be evaluated by culturing the gene-deficient epidermal keratinocytes by air-liquid interface culture to produce a cell laminate. In addition, the function of a specific gene can also be evaluated from the epidermal proliferation state or differentiation state.

Since the step of culturing gene-deficient epidermal keratinocytes by air-liquid interface culture is the same as the above-described method for producing a cell laminate of the present disclosure, a detailed description thereof will be omitted.

In the method for evaluating epidermal proliferation or differentiation of the present disclosure, for example, the epidermal proliferation state or differentiation state can be evaluated by culturing gene-deficient epidermal keratinocytes by air-liquid interface culture to produce a cell laminate, and then staining the epidermal layer with HE and observing the stained epidermal layer with an optical microscope.

In addition, in the method for evaluating epidermal proliferation or differentiation of the present disclosure, for example, the epidermal differentiation state can be evaluated by culturing gene-deficient epidermal keratinocytes by air-liquid interface culture to produce a cell laminate, and detecting gene expression in cells of the epidermal layer.

Examples of genes which are indicators of epidermal differentiation state include a loricrin gene (LOR), a periostin gene (POSTN), a transglutaminase 3 gene (TGM3), a wingless-type mouse mammary tumor virus (MMTV) integration site family member 3 gene (WNT3), a wingless-type MMTV integration site family member 4 gene (WNT4), a filaggrin gene (FLG), a kallikrein-related peptidase 14 gene (KLK14), a desmocollin 1 gene (DSC1), a caspase 14 gene (CASP14), and a fibronectin 1 gene (FN1). Among these, it is preferable to detect the expression of at least one gene selected from the group consisting of a loricrin gene, a filaggrin gene, and a caspase 14 gene and it is more preferable to detect the expression of filaggrin gene.

The method for detecting gene expression is not particularly limited. For example, the expression of RNA corresponding to the above-mentioned gene can be detected by a reverse transcription polymerase chain reaction (RT-PCR) method or a microarray method. The expression of a protein corresponding to the above-mentioned gene can also be detected by enzyme-linked immunosorbent assay (ELISA) or Western blotting.

### <Method for evaluating test substance>

The method for evaluating a test substance according to one embodiment includes a step of culturing specific gene-deficient epidermal keratinocytes (that is, the above-mentioned gene-deficient epidermal keratinocytes) in the presence of a test substance by air-liquid interface culture. Effects of a test substance on the structure, function, and the like of a cell laminate can be evaluated by culturing the gene-deficient epidermal keratinocytes in the presence of the test substance by air-liquid interface culture to produce the cell laminate.

Since the step of culturing the gene-deficient epidermal keratinocytes by air-liquid interface culture is the same as the above-mentioned method for producing a cell laminate of the present disclosure, except that the cells are cultured in the presence of a test substance, a detailed description thereof will be omitted.

The phrase "in the presence of a test substance" is not particularly limited as long as the test substance can come into contact with the gene-deficient epidermal keratinocytes. For example, a test substance may be added to the above-mentioned medium for a cell laminate. In addition, a test substance may be contained in the above-mentioned collagen-containing layer, or a cell secreting a test substance may be contained in the above-mentioned collagen-containing layer.

The test substance is not particularly limited, and may be a component contained in a pharmaceutical product or a cosmetic product, a bio-related substance such as cytokine, or another compound.

The method for evaluating a test substance according to another embodiment includes a step of bringing a test substance into contact with the above-mentioned cell laminate of the present disclosure. By bringing the test substance into contact with the cell laminate, it is possible to evaluate the effect of the toxicity or the like of the test substance on the cell laminate, the permeability of the test substance to the cell laminate, and the like.

For example, as shown in the Examples which will be described later, a cell laminate in which the epidermal layer is thinned can be obtained by culturing IGF-1R gene-deficient epidermal keratinocytes by air-liquid interface culture. Therefore, this cell laminate can be used as a thinning model or an aging model of skin for the evaluation of a test substance.

The method of bringing the test substance into contact with the cell laminate is not particularly limited, and for example, a method of applying the test substance to the epidermal layer can be mentioned.

The test substance is not particularly limited. The test substance may be a pharmaceutical product or a cosmetic product, or may be a component contained in a pharmaceutical product or a cosmetic product, or may be another compound.

### <Biological implantation material>

The biological implantation material of the present disclosure includes the above-mentioned cell laminate of the present disclosure. In the case where the biological implantation material of the present disclosure is transplanted into a diseased site or the like, the cell laminate can engraft and self-organize. At this time, even in the case where the structure or function of the cell laminate is incomplete, the cell laminate is self-organized by the action of factors *in vivo* after engraftment, whereby the object of treatment can be achieved.

Conventionally, cells derived from a patient have been cultured to produce artificial skin which is then transplanted into the patient as a biological implantation material. However, in a patient having a genetic skin disease, improvement of the disease cannot be expected even in the case where such artificial skin is transplanted. In this regard, a biological implantation material including a cell laminate produced using epidermal keratinocytes deficient in a skin disease causative gene can also be used for treating a patient having a genetic skin disease.

### EXAMPLES

Hereinafter, embodiments of the present invention will be described in more detail with reference to Examples. However, embodiments of the present invention are not limited to the following Examples unless exceeding the gist thereof.

### <Reference Example 1>

In Reference Example 1, IGF-1R gene-deficient HaCaT cells were constructed by using HaCaT cells as epidermal keratinocytes and deleting the IGF-1R gene of HaCaT cells by a CRISPR-Cas9 method.

### (1) Culture of HaCaT cells

HaCaT cells were cultured in DMEM (available from Thermo Fisher Scientific Inc.) containing 10 v/v% FBS (available from Thermo Fisher Scientific Inc.) and penicillin and streptomycin (pen/strep, available from Thermo Fisher Scientific Inc.) as a medium in a humidified incubator under conditions of 37°C and 5 v/v% CO₂. In the case where the occupancy of cells reached about 80% of the bottom area of the culture vessel (that is, 80% confluent), the passage was carried out by recovering the cells using 0.25 w/v% trypsin-ethylenediamine tetraacetic acid (EDTA) (available from Thermo Fisher Scientific Inc.). During the culture, medium exchange was carried out every 2 to 3 days.

### (2) Construction of plasmid vector for genome editing

Using a commercially available kit (Gene Art CRISPR Nuclease Vector with OFP Reporter Kit, available from Thermo Fisher Scientific Inc.), a plasmid vector for genome editing (hereinafter, also simply referred to as "plasmid" or "vector") was constructed as follows. The plasmid vector attached to this kit can express Cas9 nuclease and gRNA in a single vector and has an orange fluorescent protein (OFP) gene as a reporter gene.

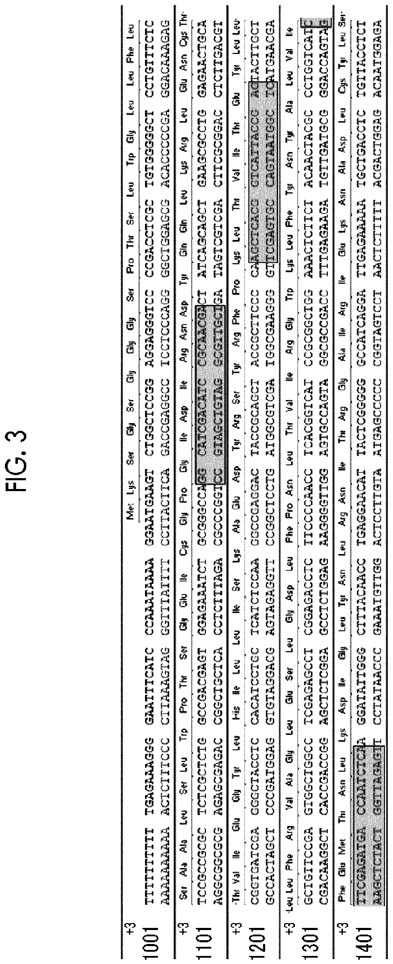

First, a target sequence on genomic DNA was selected using software such as CRISPRdirect (http://crispr.dbcls.jp/) or E-CRISP (http://www.e-crisp.org/E-CRISP/). In the case where a gene is deleted by a CRISPR-Cas9 method, it is preferable to select the target sequence from the upstream side of the gene. Therefore, from the first exon and the second exon of the IGF-1R gene, a region of 19 or 20 bases long adjacent to the proto-spacer adjacent motif (PAM) sequence was searched. Further, homology search on the genomic DNA was carried out on the searched candidate sequence, and the presence or absence of a site to be non-specifically recognized was confirmed. As a result, three types of target sequences were selected. The positions of the three target sequences on the IGF-1R gene are shown in Fig. 3. In Fig. 3, the boxed portions are the target sequences.

Based on each target sequence, three types of oligo DNAs (IGF1R. 1, IGF1R. 2, and IGF1R. 3) encoding crRNA complementary to each target sequence were designed. The base sequences of the designed oligo DNAs are shown in Table 1 below. In Table 1, the PAM sequence adjacent to each base sequence, the number of mismatched bases with the target sequence, and the gene position of the target sequence are also shown.

**[Table 1]**

| | Base sequence (5'→3') | PAM sequence | Number of mismatched bases | Gene position | SEQ ID NO |
|---|---|---|---|---|---|
| IGF1R. 1 | CTTCGAGATGACCAATCTCA | AGG | 0 | chr15[98707824] | 1 |
| IGF1R. 2 | CTCGGTAATGACCGTGAGCT | TGG | 0 | chr15[98707694] | 2 |
| IGF1R. 3 | TCGTTGCGGATGTCGATGCC | TGG | 0 | chr15[98707570] | 3 |

Subsequently, an oligo DNA was constructed by adding an additional sequence for ligation to the base sequence of each oligo DNA shown in Table 1 and the complementary sequence thereof, which was followed by denaturation at 95°C and annealing at room temperature (25°C) to construct a double-stranded oligo DNA. The constructed double-stranded oligo DNA was ligated into a vector using T4 DNA ligase attached to the kit.

Next, transformation of *E. coli* (One Shot TOP10 Chemically Competent *E. coli*) attached to the kit was carried out using the vector after ligation. The transformed *E. coli* was cultured on an agar medium supplemented with ampicillin. A part of colonized *E. coli* was recovered and the plasmid was purified using a plasmid recovery kit (available from QIAGEN, Inc.). Then, the purified plasmid was confirmed to be a desired plasmid by PCR using the U6 forward primer attached to the kit. Thereafter, the *E. coli* having a desired plasmid was cultured again, and the plasmid was purified using a plasmid recovery kit (available from QIAGEN, Inc.). The sequence of the plasmid was confirmed using a commercially available kit (BigDye Terminator v3.1 Cycle Sequencing Kit, available from ABI, "BigDye" is a registered trademark), and was used for the subsequent experiment.

### (3) Introduction of plasmid into HaCaT cells

A genome editing plasmid was introduced into HaCaT cells cultured to 70% to 80% occupancy relative to the bottom area of a 24-well plate (available from Becton, Dickinson and Company) by a lipofection method. Introduction of the plasmid was carried out using a Lipofectamine 3000 reagent (available from Thermo Fisher Scientific Inc., "Lipofectamine" is a registered trademark) according to the manufacturer's protocol. The medium was exchanged after 48 hours of introduction of the plasmid, and dead cells were removed. In the case where the OFP-positive cells were confirmed with a fluorescence microscope (excitation wavelength: 548 nm, fluorescence wavelength: 560 nm), the plasmid introduction rate was about 2% to 3%.

### (4) Cell enrichment by fluorescence activated cell sorting (FACS)

HaCaT cells after introduction of the plasmid were recovered using 0.25 w/v% trypsin-EDTA (available from Thermo Fisher Scientific Inc.), and then OFP-positive cells were enriched with a cell sorter (BD FACSAria, available from Becton, Dickinson and Company, "FACSAria" is a registered trademark). In the case where the separated OFP-positive cells were observed with a fluorescence microscope (excitation wavelength: 548 nm, fluorescence wavelength: 560 nm), the plasmid introduction rate was about 100%.

### (5) Confirmation of mutation in IGF-1R gene

To detect the presence or absence of a mutation in the IGF-1R gene, Cell assay was carried out using a special enzyme (Cell endonuclease) that recognizes and cleaves the mismatched portion of heteroduplex DNA. The principle of the Cell assay is as follows. The region containing the target sequence on the genomic DNA is amplified by PCR, and the amplified product is denatured and then re-annealed. In the case where a mutation is present in the amplified product, heteroduplex DNA is formed upon re-annealing, so the mismatched portion is cleaved by Cell endonuclease. The presence or absence of a gene mutation can be confirmed by confirming the presence or absence of this cleavage by electrophoresis.

The Cell assay was carried out as follows using a commercially available kit (GeneArt Genomic Cleavage Detection Kit, available from Thermo Fisher Scientific Inc.). The base sequences of forward primer (hIGF-1R_E2_13F) and reverse primer (hIGF-1R_E2_401R) used for PCR are shown in Table 2 below.

**[Table 2]**

| | Base sequence (5'→3') | SEQ ID NO |
|---|---|---|
| hIGF-1R_E2_13F | GCATCGACATCCGCAACGAC | 4 |
| hIGF-1R_E2_401R | GGACACCGCATCCAGGATCA | 5 |

Genomic DNA was extracted from FACS-enriched OFP-positive cells and the region containing the target sequence of the IGF-1R gene was amplified using the above-mentioned primers. The amplified product was denatured at 95°C and then re-annealed at room temperature (25°C) to obtain double-stranded DNA. Cell endonuclease was added to the double-stranded DNA after re-annealing, followed by incubation at 37°C for 1 hour, and then the enzyme was inactivated by heat. Then, the amplified product after the enzymatic reaction was electrophoresed on 4 w/v% agarose gel (available from Wako Pure Chemical Industries, Ltd.), and the electrophoretic pattern was confirmed. As a result, three bands were detected, thus confirming the introduction of mutation into the IGF-1R gene.

### (6) Cell cloning

FACS-enriched OFP-positive cells were diluted to a density of 0.5 cells/well to 1 cell/well and seeded in a 96-well plate. After culturing the cells for 2 weeks to 4 weeks, the genomic DNA of the proliferated cells was extracted and subjected to the Cell assay in the same manner as described above. As a result, mutation of the IGF-1R gene was confirmed for clones of 24 strains.

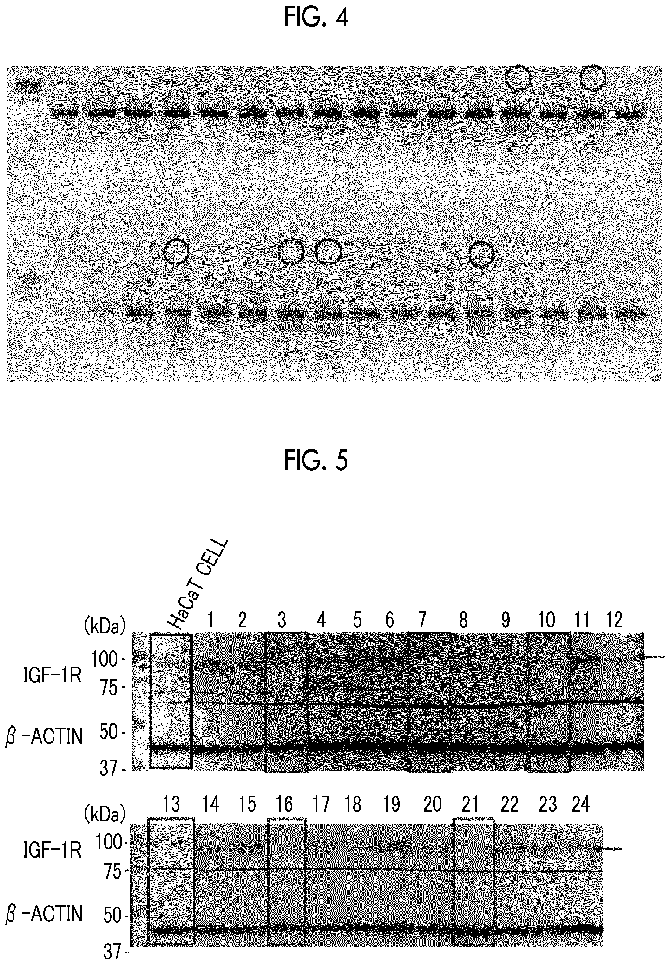

Apart of the electrophoretic pattern in the Cell assay is shown in Fig. 4. In Fig. 4, the lanes marked with a circle correspond to clones in which mutation of the IGF-1R gene was confirmed.

### (7) Confirmation of deficiency of IGF-1R gene (Western blotting)

The Cell assay-positive strain after cloning was cultured, the cells were solubilized with a radio-immunoprecipitation assay (RIPA) buffer (available from Thermo Fisher Scientific Inc.), and then the protein was quantitated using a commercially available kit (BCA Protein Assay Kit, available from Thermo Fisher Scientific Inc.). For each clone, 20 µg of protein was developed on a 10 w/v% polyacrylamide gel and the protein was transferred to a polyvinylidene difluoride (PVDF) membrane using a blotting apparatus (available from ATTO Corp.). Thereafter, an IGF-1R protein was detected using an IGF-1R-specific antibody (available from Thermo Fisher Scientific Inc.). For positive control, HaCaT cells not transfected with plasmid were used. As a result, the IGF-1R protein was not detected and the deficiency of the IGF-1R gene was confirmed in clones of 6 strains in 24 strains. The clone in which the deficiency of the IGF-1R gene was not recognized despite the confirmation of the mutation of the IGF-1R gene is presumed to have not caused a frame shift due to the gene mutation.

The results of Western blotting are shown in Fig. 5. In Fig. 5, lane 3, lane 7, lane 10, lane 13, lane 16, and lane 21 correspond to clones of 6 strains in which the deficiency of the IGF-1R gene was recognized.

Among the clones of these 6 strains, the clone of lane 7 (hereinafter, also referred to as "IGF-1R-deficient HaCaT cell 1") and the clone of lane 10 (hereinafter, also referred to as "IGF-1R-deficient HaCaT cell 2") were used for the subsequent experiments. In addition, the clone of lane 15, in which the deficiency of the IGF-1R gene was not recognized, was used as a Sham control cell. Hereafter, in all operations, the clone of lane 15 was used as a Sham control.

### (8) Confirmation of deficiency of IGF-1R gene (cell immunostaining)

IGF-1R-deficient HaCaT cells 1 and Sham control cells were respectively treated with phosphate buffered saline (PBS) containing 4 w/v% paraformaldehyde (20 g of paraformaldehyde dissolved in 500 mL of 0.1 mol/L PBS (pH 7.4) (available from Wako Pure Chemical Industries, Ltd.)) for 15 minutes to fix the cells. The cells were washed twice with PBS and subjected to a permeation treatment by treating with PBS containing 0.1 v/v% Triton-X100 for 20 minutes. Then, blocking was carried out by treating the cells with a blocking agent (Blocking One, available from Nacalai Tesque, Inc.) for 1 hour or more. Thereafter, a 200-fold diluted solution of anti-IGF-1R antibodies (available from Thermo Fisher Scientific Inc.) was added thereto, followed by reaction at 4°C overnight. The cells were washed about three times with PBS and then reacted for 2 hours with a 200-fold diluted solution of anti-mouse-fluorescein isothiocyanate (FITC) antibodies (available from Abcam PLC). The cells were washed about three times with PBS, nuclear stained with Hoechst (available from Dojindo Laboratories Co., Ltd.), encapsulated and observed with a fluorescence microscope (available from Keyence Corporation).

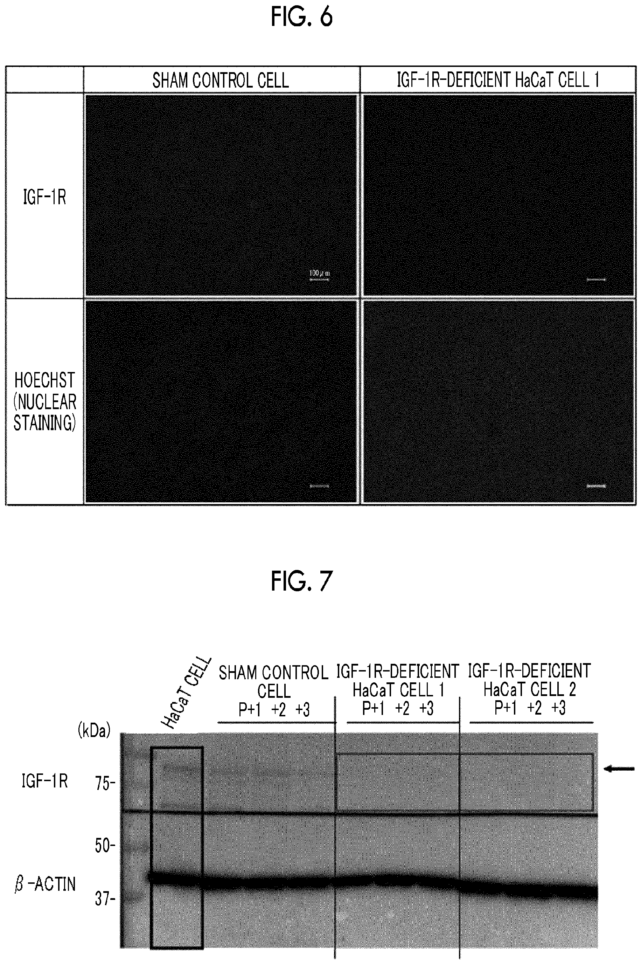

The results of cell immunostaining are shown in Fig. 6. The scale bar in the figure shows 100 µm. As can be seen from Fig. 6, an IGF-1R protein was confirmed in Sham control cells, whereas IGF-1R protein was not confirmed in IGF-1R-deficient HaCaT cells 1.

### (9) Confirmation of continuity of deficiency of IGF-1R gene

IGF-1R-deficient HaCaT cells 1, IGF-1R-deficient HaCaT cells 2, and Sham control cells were additionally subcultured and the IGF-1R protein was detected by Western blotting in the same manner as described above. As positive control, HaCaT cells not transfected with plasmid were used.

The results of Western blotting are shown in Fig. 7. In Fig. 7, "P" indicates the number of passages before the experiment, "P+1" means that the cells were additionally passaged once, "P+2" means that the cells were additionally passaged twice, and "P+3" means that the cells were additionally passaged three times. As can be seen from Fig. 7, the deficiency of the IGF-1R gene was continued even in the case where the passages of IGF-1R-deficient HaCaT cells 1 and IGF-1R-deficient HaCaT cells 2 were repeated.

### (10) Confirmation of sequence of IGF-1R gene

Genomic DNA was extracted from IGF-1R-deficient HaCaT cells 1 and the sequence of the region containing the target sequence of the IGF-1R gene was confirmed by a conventional method.

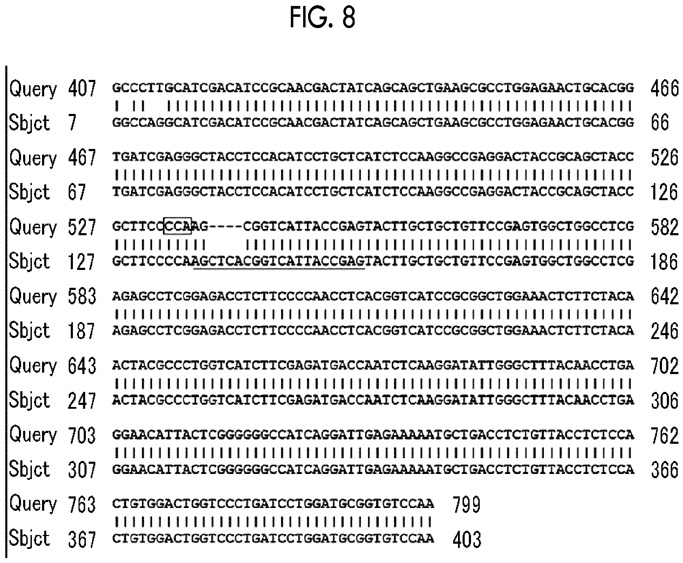

The results of confirming the sequence are shown in Fig. 8. In Fig. 8, the upper sequence shows the sequence of the region containing the target sequence of the IGF-1R gene in IGF-1R-deficient HaCaT cells 1 and the lower sequence shows the original sequence in the same region. The underlined sequence corresponds to the target sequence and the boxed sequence corresponds to the PAM sequence. As can be seen from Fig. 8, it was confirmed that 4 bases (CTCA) in the target sequence were deleted in the IGF-1R-deficient HaCaT cells 1.

### <Example 1>

In Example 1, IGF-1R-deficient HaCaT cells 1 were cultured on a polycarbonate support by air-liquid interface culture to produce a cell laminate.

### (1) Preparation of medium for cell laminate

294.04 mg of CaCl₂·2H₂O was dissolved in 1 mL of ultrapure water, stirred with a vortex mixer, and then filter-sterilized. The obtained CaCl₂ solution was dispensed into a microtube and stored at -30°C until use.

A DMEM containing 10 v/v% FBS as a medium for normal human dermal fibroblasts and a medium for normal human epidermal keratinocytes (HuMedia-KG 2, Kurabo) excluding a human epidermal growth factor (hEGF) were mixed at a ratio of 1:1 (volume ratio). 225 µL of the above-prepared CaCl₂ solution (final concentration of calcium in the medium: 1.8 mmol/L) was added to 500 mL of the obtained medium. The medium after the addition of the CaCl₂ solution was dispensed into a 50 mL FALCON tube (available from Corning Inc., "FALCON" is a registered trademark), and ascorbic acid 2-glucoside was added so as to have a concentration of 0.1 w/w%, whereby a medium for a cell laminate was prepared.

### (2) Production of cell laminate

IGF-1R-deficient HaCaT cells 1 were dispersed at a cell concentration of 2×10⁵ cells/0.2 mL in a medium for a cell laminate to prepare a cell dispersion liquid. 0.2 mL of the obtained cell dispersion liquid was seeded on a polycarbonate cell culture insert (available from Becton, Dickinson and Company), and the cell culture insert was placed in wells of a 12-well plate. 1.5 mL of the medium for a cell laminate was added to the outside of the cell culture insert and the cells were cultured for 24 hours under the conditions of 37°C and 5 v/v% CO₂. After 24 hours, the medium in the cell culture insert was gently removed to form an air-liquid interface, and the cells were cultured for another 14 days. The medium exchange was carried out every two days.

### (3) Histological analysis of cell laminate (HE staining)

The cell laminate obtained by culturing for 14 days was fixed using PBS containing 4 w/v% paraformaldehyde (20 g of paraformaldehyde dissolved in 500 mL of 0.1 mol/L PBS (pH 7.4)) (available from Wako Pure Chemical Industries, Ltd.). The fixed cell laminate was immersed in 0.1 mol/L PBS and the liquid was exchanged several times to replace the fixing liquid. The cell laminate was cut into a strip shape by a razor and embedded in paraffin. Then, a section having a thickness of 4 µm was prepared using a microtome, attached to a slide glass and dried at 45°C. The obtained paraffin-embedded section of the cell laminate was stored at room temperature (25°C).

The paraffin-embedded section was deparaffinized with xylene and then sequentially immersed in aqueous ethanol solutions whose concentrations were gradually decreased from 100 v/v% to 70 v/v%. The sample was washed with distilled water, immersed in a hematoxylin staining liquid (available from Wako Pure Chemical Industries, Ltd.) for 5 minutes and washed with running water. Further, the sample was immersed in 70 v/v% ethanol containing 0.2 w/v% HCl for 1 second and washed with running water for 10 minutes. Subsequently, the sample was immersed in an eosin staining liquid (available from Wako Pure Chemical Industries, Ltd.) for 10 minutes, sequentially immersed in aqueous ethanol solutions whose concentrations were gradually increased from 95 v/v% to 100 v/v% for dehydration, and then immersed in xylene for 2 minutes to 3 minutes. An encapsulating agent was placed on the obtained sample which was then covered with a cover glass, dried and encapsulated.

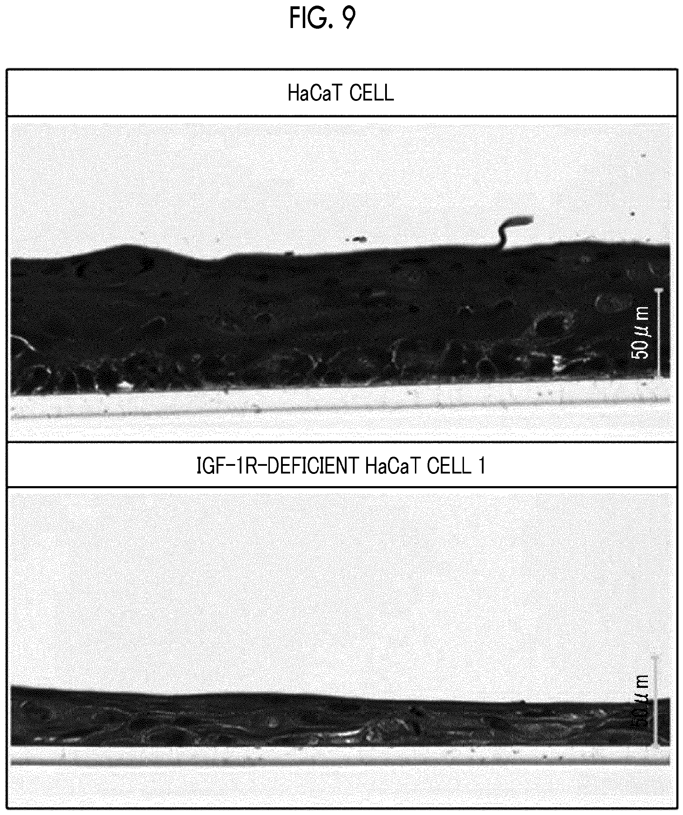

Fig. 9 shows HE stained images of cell laminates obtained by culturing IGF-1R-deficient HaCaT cells 1 and control HaCaT cells. The scale bar in the figure shows 50 µm. As can be seen from Fig. 9, epidermal proliferation and differentiation were significantly decreased in the cell laminate obtained by culturing the IGF-1R-deficient HaCaT cells 1, as compared with the cell laminate obtained by culturing HaCaT cells.

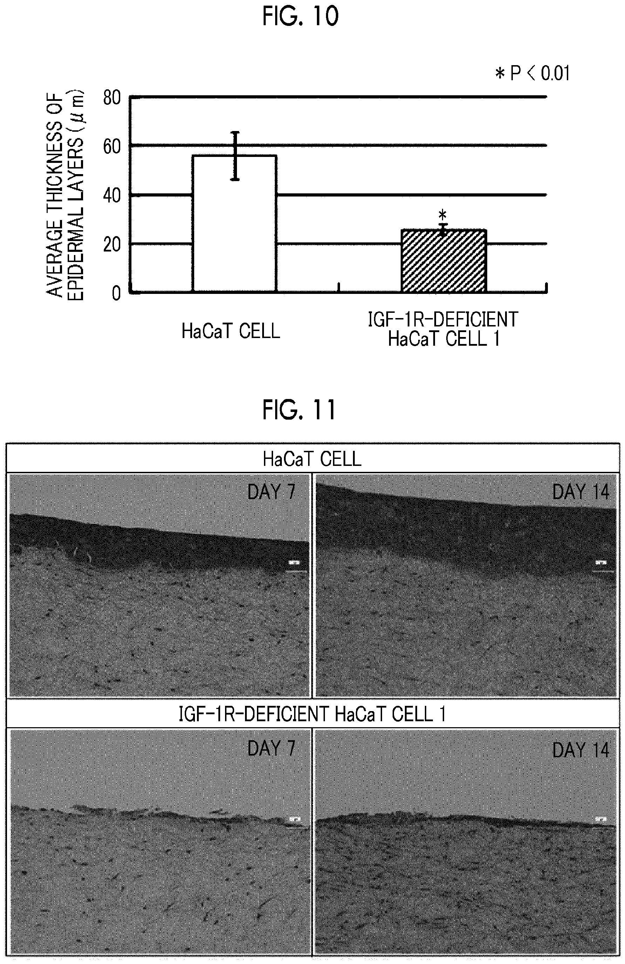

Fig. 10 shows an average thickness of the epidermal layers of the cell laminates obtained by culturing IGF-1R-deficient HaCaT cells 1 and control HaCaT cells (n = 3 in each case). As can be seen from Fig. 10, the average thickness of the epidermal layers was about 55 µm in the cell laminates obtained by culturing HaCaT cells, whereas the average thickness of the epidermal layers was about 25 µm in the cell laminates obtained by culturing IGF-1R-deficient HaCaT cells 1, in which the epidermal layer was significantly thinned (P<0.01).

### <Example 2>

In Example 2, IGF-1R-deficient HaCaT cells 1 were cultured on a collagen gel (support layer) in which fibroblasts were embedded by air-liquid interface culture to produce a cell laminate.

### (1) Construction of collagen gel in which fibroblasts are embedded

5 mL of a medium for a cell laminate (prepared in Example 1) containing a total of 5×10⁵ normal human neonatal foreskin dermal fibroblasts (available from Kurabo Industries Ltd.) and acid-solubilized type I collagen (derived from bovine) having a final concentration of 1 mg/mL was dispensed as a collagen solution into a 6-well plate. Then, under the conditions of 37°C and 5 v/v% CO₂, the cells were cultured in static culture or while stirring at a rate of 5 revolutions per minute to 60 revolutions per minute. After culturing for 2 days to 3 days, a collagen gel in which the bottom surface contracted to about 1.0 cm in diameter was obtained.

### (2) Production of cell laminate

IGF-1R-deficient HaCaT cells 1 were dispersed at a cell concentration of 2×10⁵ cells/0.2 mL in a medium for a cell laminate to prepare a cell dispersion liquid. A ring (inner diameter: 7 mm) was placed on the collagen gel, and the medium inside and outside the ring was removed. 0.4 mL of the cell dispersion liquid was added into the ring, and it was confirmed that the cell dispersion liquid did not leak to the outside of the ring. Next, 3 mL of the medium for a cell laminate was added to the outside of the ring and the cells were cultured for 24 hours under the conditions of 37°C and 5 v/v% CO₂.

After 24 hours, the medium inside and outside the ring was removed while paying attention not to break the layer of the IGF-1R-deficient HaCaT cells 1 layered on the collagen gel, and the ring was removed using tweezers. A medium for a cell laminate was then added from the edge of the well. At that time, the amount of the medium was adjusted to about 2 mL to 3 mL so that the boundary between the IGF-1R-deficient HaCaT cells 1 and the collagen gel was not immersed in the medium so as to bring the medium into contact with the IGF-1R-deficient HaCaT cells 1. From this day air-liquid interface culture was started and the cells were cultured for 7 days or 14 days. The medium exchange was carried out every two days.

### (3) Histological analysis of cell laminate (HE staining)

The cell laminate obtained by culturing for 7 days or 14 days was subjected to HE staining in the same manner as in Example 1.

Fig. 11 shows HE stained images of cell laminates obtained by culturing IGF-1R-deficient HaCaT cells 1 and control HaCaT cells. The scale bar in the figure shows 50 µm. As can be seen from Fig. 11, epidermal proliferation and differentiation were significantly decreased in the cell laminate obtained by culturing the IGF-1R-deficient HaCaT cells 1, as compared with the cell laminate obtained by culturing HaCaT cells.

Comparing Fig. 9 with Fig. 11, it is found that the epidermal layer is thinner in the case of culturing on a collagen gel in which fibroblasts are embedded than in the case of culturing on a polycarbonate support. This is presumed to be due to the fact that it is more difficult for IGF-1R-deficient HaCaT cells 1 to come into contact with the medium for a cell laminate in the case of culturing on a collagen gel in which fibroblasts are embedded.

The disclosure of JP2016-150296 filed on July 29, 2016 is hereby incorporated by reference in its entirety.

All documents, patent applications, and technical standards described in the present specification are incorporated herein by reference to the same extent as the case where each individual document, patent application, and technical standard were specifically and individually indicated to be incorporated by reference.

### [Sequence Listing]

International Application No. FS-F06741 under Patent Cooperation Treaty-CELL LAMINATE, METHOD FOR PRODUCING CELL LAMINATE, JP17024647 20170705----00130142651701419836 Normal 20170705110358201702280912248570_P1AP101_FS_2.app

## Claims

1. A cell laminate comprising:
an epidermal layer in which specific gene-deficient epidermal keratinocytes are laminated.

2. The cell laminate according to claim 1, wherein the epidermal keratinocyte is a human epidermal keratinocyte.

3. The cell laminate according to claim 1 or 2, further comprising:
a support layer.

4. The cell laminate according to claim 3, wherein the support layer is a layer including collagen and at least one of mesenchymal cells or mesenchymal stem cells.

5. The cell laminate according to any one of claims 1 to 4, wherein the specific gene includes a cell membrane receptor gene.

6. The cell laminate according to any one of claims 1 to 5, wherein the specific gene includes an insulin-like growth factor-1 receptor gene.

7. A method for producing a cell laminate, comprising:
a step of culturing specific gene-deficient epidermal keratinocytes by air-liquid interface culture.

8. The method for producing a cell laminate according to claim 7, wherein the epidermal keratinocyte is a human epidermal keratinocyte.

9. The method for producing a cell laminate according to claim 7 or 8, further comprising:
a step of deleting the specific gene of the epidermal keratinocyte using a site-specific nuclease.

10. The method for producing a cell laminate according to any one of claims 7 to 9, wherein the step of culturing specific gene-deficient epidermal keratinocytes by air-liquid interface culture includes culturing the specific gene-deficient epidermal keratinocytes on a support layer by air-liquid interface culture.

11. The method for producing a cell laminate according to claim 10, wherein the support layer is a layer including collagen and at least one of mesenchymal cells or mesenchymal stem cells.

12. The method for producing a cell laminate according to any one of claims 7 to 11, wherein the specific gene includes a cell membrane receptor gene.

13. The method for producing a cell laminate according to any one of claims 7 to 12, wherein the specific gene includes an insulin-like growth factor-1 receptor gene.

14. A method for evaluating epidermal proliferation or differentiation, comprising:
a step of culturing specific gene-deficient epidermal keratinocytes by air-liquid interface culture.

15. A method for evaluating a test substance, comprising:
a step of culturing specific gene-deficient epidermal keratinocytes in the presence of a test substance by air-liquid interface culture.

16. A method for evaluating a test substance, comprising:
a step of bringing a test substance into contact with the cell laminate according to any one of claims 1 to 6.

17. A biological implantation material comprising:
the cell laminate according to any one of claims 1 to 6.


SEQUENCE LISTING

<110>  FUJIFILM CORPORATION

<120>  CELL LAMINATE AND METHOD FOR PRODUCING THE SAME

<130>  FS-F06741-00

<150>  JP2016-150296
<151>  2016-07-29

<160>  5

<210>  1
<211>  20
<212>  DNA
<213>  Artificial Sequence

<220>
<223>  Oligonucleotide designed based on the target sequences of IGF-1R gene

<400>  1
cttcgagatg accaatctca                                                   20


<210>  2
<211>  20
<212>  DNA
<213>  Artificial Sequence

<220>
<223>  Oligonucleotide designed based on the target sequences of IGF-1R gene

<400>  2
ctcggtaatg accgtgagct                                                   20


<210>  3
<211>  20
<212>  DNA
<213>  Artificial Sequence

<220>
<223>  Oligonucleotide designed based on the target sequences of IGF-1R gene

<400>  3
tcgttgcgga tgtcgatgcc                                                   20


<210>  4
<211>  20
<212>  DNA
<213>  Artificial Sequence

<220>
<223>  Forward primer for amplification of IGF-1R gene

<400>  4
gcatcgacat ccgcaacgac                                                   20


<210>  5
<211>  20

<212>  DNA
<213>  Artificial Sequence

<220>
<223>  Reverse primer for amplification of IGF-1R gene

<400>  5
ggacaccgca tccaggatca                                                    20